(19) Europäisches Patentamt
European Patent Office
Office européen des brevets

(11) **EP 2 896 356 A1**

(12) **EUROPEAN PATENT APPLICATION**

(43) Date of publication:
**22.07.2015 Bulletin 2015/30**

(21) Application number: **14195630.0**

(22) Date of filing: **22.12.2010**

(51) Int Cl.:
*A61B 5/00* [(2006.01)]    *A61B 5/024* [(2006.01)]
*A61B 5/0245* [(2006.01)]    *A61B 5/0488* [(2006.01)]
*A61B 5/08* [(2006.01)]

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.12.2009 EP 09180610**

(62) Document number(s) of the earlier application(s) in
accordance with Art. 76 EPC:
**10798336.3 / 2 515 747**

(71) Applicant: **DELTA, Dansk Elektronik, Lys &
Akustik
2970 Hørsholm (DK)**

(72) Inventors:
• **Thomsen, Erik V.
3540 Lynge (DK)**

• **Haahr, Rasmus Grønbek
2820 Gentofte (DK)**
• **Duun, Sune
3520 Farum (DK)**
• **Hoppe, Karsten
2100 Copenhagen Ø (DK)**
• **Branebjerg, Jens
2970 Hørsholm (DK)**

(74) Representative: **Nielsen, Leif
Patrade A/S
Fredens Torv 3A
8000 Aarhus C (DK)**

Remarks:
This application was filed on 01-12-2014 as a
divisional application to the application mentioned
under INID code 62.

(54) **A monitoring system**

(57) The present invention relates to a novel monitoring system suitable for attachment to a surface of a subject and for monitoring physiological signals of a subject wearing the system.

Fig. 6

EP 2 896 356 A1

**Description**

The present invention relates to a novel monitoring system suitable for attachment to a surface of a subject and for monitoring physiological signals of a subject wearing the system.

BACKGROUND OF THE INVENTION

WO 2006094513 discloses a micro electronic systems predominantly for monitoring physiological or neurological conditions. The system is embedded in a three-dimensional adhesive device which can be attached to the skin of a mammal. The microelectronic system use wireless communication and it is useful for measuring ECG (Electro CardioGraphy), EMG (Electro MyoGraphy), EEG (Electro EncephaloGraphy), blood glucose, pulse, blood pressure, pH, and oxygen.

WO 03/065926 discloses a wearable biomonitor with a flexible and thin integrated circuit. The disclosure includes ways to achieve high comfort of wear by using a thin layer adhesive or pads of adhesive for fixation to the skin.

US 5273036 relates to an apparatus for monitoring respiration comprising a photoplethysmographic sensor.

US 5458124 discloses electro-cardiographic-electrodes being attached to the body by double-sided pressure sensitive adhesive.

US 6372951 discloses a sensor operatively connected to a disposable article, fitted to the wearer by an adhesive patch. A wide variety of body adhering compositions may be used.

US 6385473 discloses a laminated sensor device attached to mammalian subject with two strips of hydrocolloid adhesive. The laminated structure consists also of hydrogel in contact with hydrocolloid adhesive.

WO 9959465 discloses an apparatus for monitoring the physiological condition of a patient.

US 5054488 discloses an opto-electronic sensor for producing electrical signals representative of a physiological condition. The sensors may be attached to the body by a double-sided pressure sensitive adhesive on a polyester lining.

Rasmus G. Haahr et al. Proceedings of the 5th International Workshop on Wearable and Implantable Body Sensor Networks, in conjunction with The 5th International Summer School and Symposium on Medical Devices and Biosensors The Chinese University of Hong Kong, HKSAR, China. Jun 1-3, 2008, relates to a wearable for Wireless continuous monitoring of physiological signals in chronically diseased patients.

Sune Duun et al. IEEE SENSORS 2007 Conference describes a photodiode for reflectance pulse oximetry in wireless applications of a patch.

Rasmus G. Haahr et al. Proceedings of the 29th Annual International Conference of the IEEE EMBS Cite Internationale, Lyon, France August 23-26, 2007 describes a photodiode for reflectance pulse oximetry in wireless applications of a patch.

US 2009/0076343 discloses a heart failure management system having an adherent device comprising an energy management device and a wireless communication device. The adherent device further comprises a core sensor and a second sensor, wherein the second sensor is activated upon the core sensor raises a flag. Said second sensor is used to verify the physiological signal from the core sensor.

WO 2006/109072 discloses a wearable monitoring device comprises three ECG channels for measuring different physiological signals of the subject in real time, wherein the physiological signal measured by a first ECG channel is used to switch on the two other ECG channels if the measurement of an additional physiologic signal is required.

OBJECT OF THE INVENTION

It is an object of embodiments of the invention to provide an "intelligent" monitoring system or device, which system is attached to the surface of a subject in need of monitoring and which system may provide an output of data limited to the most critical and essential physiological parameters of the subject and with the lowest consumption of time and/or power.

It is to be understood that the measuring of the most critical and essential physiological parameters of the subject may be time and power consuming and may only be needed under certain physical or physiological conditions. Further, the measuring of the most critical and essential physiological parameters will provide the person receiving the output from the monitoring system with data of higher quality, which will enable this person to better take the necessary and needed action, such as an immediate medical treatment.

SUMMARY OF THE INVENTION

It has been found by the present inventor(s) that the device according to the present invention solve the problem of high power consumption and redundant data output from monitoring systems by having the measurement of a first signal, such as a low power consuming signal, to control or trigger the measurement of a second more critical and essential physiological signal.

So, in a first aspect the present invention relates to a minimal invasive monitoring system suitable for attachment to a surface of a subject, the system comprising at least one first sensor which can receive a first signal and at least one second sensor which can receive a second physiological signal from the subject, the sensors being controlled by a microelectronic system being wearable by the subject, powered by independent powering, and comprising a communication structure optionally for wireless transfer of monitoring data, wherein the monitoring data based on the second physiological signal is under control of the monitoring data based on the first signal.

In a second aspect the present invention relates to a system comprising the monitoring system according to the present invention, and a data processing unit receiving monitoring data from the monitoring system and operating an algorithm based on the monitoring data from the first and second sensor to provide an output that control the monitoring data of the second sensor, or an output indicating the state of at least one physiological parameter of a subject carrying the monitoring system. In some embodiments the data processing unit is an integral part of the microelectronic system of the monitoring system. However, in alternative embodiments, the data processing unit is placed in another location, such as in a hospital, and is receiving the monitoring data of the monitoring system through the wireless communication system.

In a third aspect the present invention relates to a method for monitoring at least one physiological parameter of a subject, wherein a monitoring system according to present invention is placed on the surface of a subject and data from a system according to the present invention provide an output indicating the state of at least one physiological parameter of a subject carrying the monitoring system.

LEGENDS TO THE FIGURE

Fig. 1 is an illustration of an electronic patch with a photoplethysmographic sensor. The sensor consists of commercial LEDs and a specially designed ring shaped photodiode. Besides the photophethysmographic sensor the electronic patch also contains electronics for signal processing, wireless radio communication and coin cell battery which can power the patch for a period of one week. These components are embedded in a hydrocolloid adhesive material. The patch has a size of 88 mm by 60 mm and is 5 mm thick.

Fig. 2. Ring shaped photodiode with LEDs in the center mounted on bottom side of PCB.

Fig. 3 is the top side of the printed circuited board (PCB) showing the types of electronic components which is utilized in the pulse oximetry version of the Electronic Patch.

Fig. 4. CAD drawing of the parts in the electronic patch and how they are assembled.

Fig. 5. The assembled patch with a pulse oximetry sensor made as a concentric photodiode around two LEDs placed in the center. The little square frame around the LEDs is to prevent light going directly from the LEDs into the photodiode.

Fig. 6 shows two photoplethysmograms measured at the sternum.

Fig. 7 shows an ECG measurement using 3-leads, standard wet electrodes, and wire connection to a standard patient monitor.

Fig. 8: PPG measured on the finger using a transmission probe and a standard patient monitor comprising a pulse oximeter.

Fig. 9: Measurement of respiration by the fraction of the $CO_2$ in the airflow by a standard patient monitor.

Fig. 10: PPG (infrared wavelength of light) measured at the sternum by an annular reflectance probe embedded in a 3-dimensional adhesive patch.

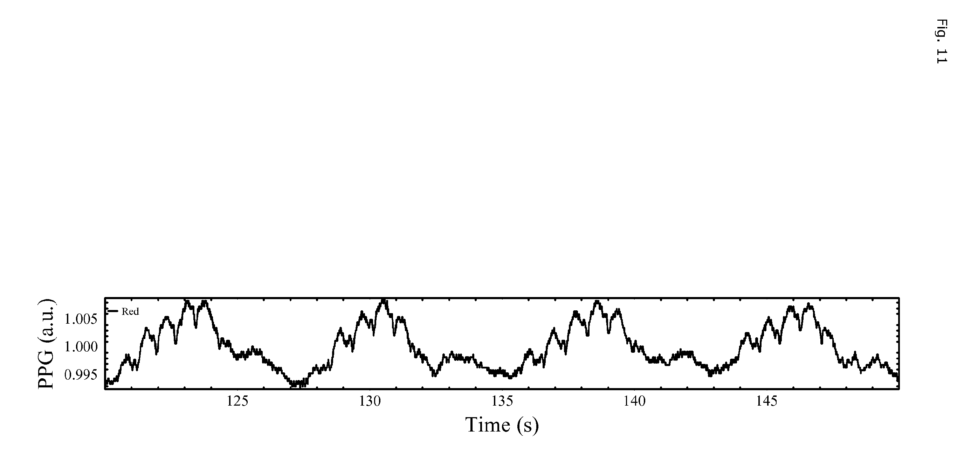

Fig. 11: PPG (red wavelength of light) measured at the sternum by an annular reflectance probe embedded in a 3-dimensional adhesive patch.

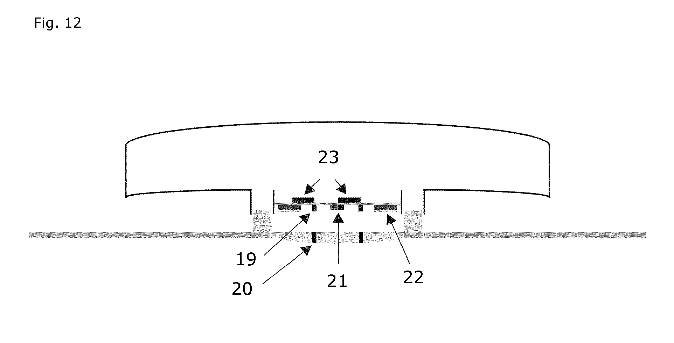

Fig. 12 illustrates a possible integration of the optical system and components in the monitoring device. The optical components are integrated as a part of the Processor. The optical signals are guided using the Transmission Structures to the Data Collector and further into the tissue through the hydro gel. Herein, numeral 19 refers to a Light shielding on PCB, numeral 20 refers to light shielding in gel, numeral 21 refers to LEDs, numeral 22 refers to photodiodes, and numeral 23 refers to amplifier circuits.

Fig. 13 illustrates a possible integration of the optical system and components in the monitoring device. The optical components are integrated as a part of the Data Collector. The Data Collector and Processor have electrical connections through the Transmission Structures by conduction silicon wires. Herein, numeral 24 refers to a light shielding, numeral 25 refers to LEDs, numeral 26 refers to photodiodes, numeral 27 refers to a coin cell battery, and numeral 28 refers to amplifier circuits.

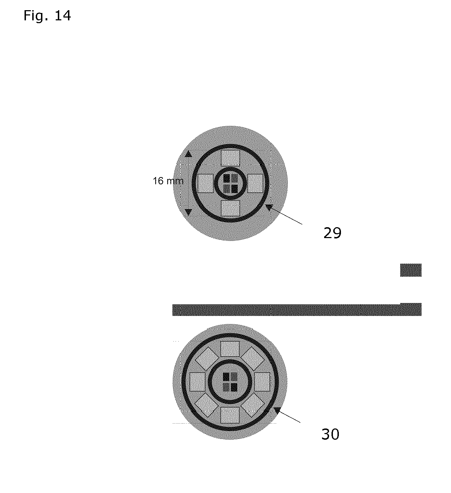

Fig. 14 shows the top view of two layouts of a printed circuit board with electro optic components of light emitting diodes (LEDs) and photodiodes. 4 - 8 photodiodes are mounted in an annular geometry with light emitting diodes (LEDs) in the centre. The wavelengths of the LEDs are 660 nm and 940 nm, respectively. The photodiodes are e.g. the BPW34 or similar. Herein, numeral 29 and 30 refer to shieldings.

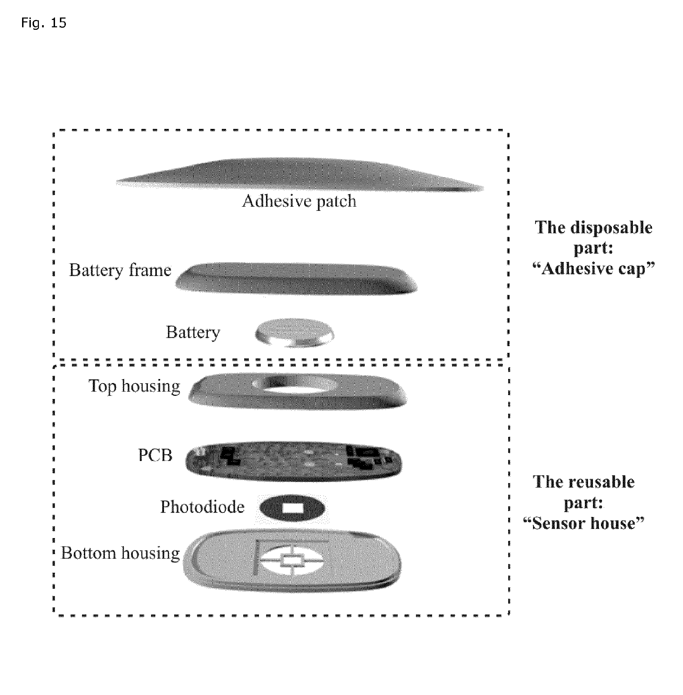

Fig. 15 shows an illustration of a 3-dimensionally structured patch illustrating the encapsulation of an optical sensor system for measuring the respiration rate by optical methods.

## DETAILED DISCLOSURE OF THE INVENTION

As described above the present invention describes a monitoring system suitable for attachment to a surface, such as the skin of a subject, such as a human, which system at least comprises one or more sensors, a microelectronic system to control sensors, powering means, and a communication structure optionally for wireless transfer of monitoring data.

The term "subject" as used herein refers to any human or animal, such as mammals, that requires or benefit from being monitored with the system or device according to the present invention. The term includes but is not limited to patients, such as hospitalized patients, human professionals, such as military persons, firemen, domestic animals, such as dogs, cats, cows, pigs, goats, and horses.

The system has to comprise at least one first sensor, which can receive a first signal, and at least one second sensor, which can receive a second physiological signal from the subject having the system attached, which second physiological signal is different from the first signal. It is to be understood that the first and second sensor may be contained within the same physical sensor, if a sensor element is able to receive two or more different signals.

Accordingly, in some embodiments, the first and second sensor is same sensor element. In other embodiments, the first signal and the second physiological signal are received by different sensors of the monitoring system. It is to be understood that the system according to the present invention may comprise 1, 2, 3, 4, 5, 6, 7, 8, 9, 10, or more sensors, that are able to obtain 1, 2, 3, 4, 5, 6, 7, 8, 9, 10, or more signals, such as physiological or non-physiological signals.

The sensors may be selected from a wide variety of different sensors; each specialized in receiving different signals for the monitoring of different physical and physiological parameters relevant to the subject having the system attached on its surface.

The system may in some embodiments, includes sensor(s) for the optical measurement based on photoplethysmography (PPG) to measure the respiration, comprising light source(s) and photo detector.

As used herein "respiration" refers to any physiological parameter in relation to respiration, such as just a positive indication of a process of respiration or not, respiration frequency, as well as physiological signals, such as from photoplethysmograms (PPG) representing respiration. In some embodiments "respiration" refers to the comparison of a photoplethysmogram (PPG) representing respiration from a subject with a reference photoplethysmogram. A reference photoplethysmogram may be from a population of disease individuals with a specific indication or alternatively from a population of normal individuals. In still another embodiment, the reference is from the subject having the system attached, but under different or previous conditions, such as under normal conditions.

In some embodiments "respiration" refers to respiration frequency.

The system is configured to be worn on the body, e.g. to the sternum for efficient measurement of respiration and physiological parameters measured on the heart. The system may be combined with further technical features, e.g. measurements of other physiological parameters like arterial oxygen saturation ($SpO_2$) by pulse oximetry, heart function, heart beat rate, and pulse.

For measuring the respiration at least one light source is used e.g. a light emitting diode in any suitable range of the electromagnetic spectrum, such as in the red to infrared range. To detect the optical signal at least one photo detector e.g. a photodiode is used. The optical signal is modulated inside the tissue by the physiology of the body, and by analyzing this optical signal returning from the inside of the tissue various physiological parameters can be calculated.

The configuration between light source(s) and light detector(s) may be a specific shape such as for example side by side or a ring-shape, where an annular photo detector where the light sources are placed in the middle of the surrounding photoactive area.. The design and configuration between light source and light detector are important parameters which impact the quality of the optical signal.

To combine measurement of the respiratory frequency with measurement of the arterial oxygen saturation ($SpO_2$) and heart function, heart beat rate, pulse, pulse oximetry is suitably employed. In pulse oximetry at least two wavelengths must be used, typically one in the red and one in the near infrared range. For example, by alternating on and off of the light sources and read off the photo detector in a sequence, e.g. red on, read photo detector, red off, infrared on, read photo detector, infrared off, two photoplethysmograms is measured. These photoplethysmograms measured on the sternum looks differently from photoplethysmograms measured on other locations on the body e.g. the finger. This is due to the respiratory information contained in the signal. A number of mathematical methods can be used to calculate the $SpO_2$ e.g. Discrete Saturation Transform (DST) by Masimo Corporation or Independent Component Analysis (ICA). The respiratory frequency and heart beat rate, pulse can be found from either of the two photoplethysmograms, e.g. by time and frequency domain analysis.

The system, such as contained within a patch, may as described elsewhere, contain a micro controller or micro processor for controlling the measuring sequence, signal processing, and calculation of physiological parameters from monitoring data, such as a photoplethysmograms. Furthermore, wireless technologies are contained in the system enabling wireless transmission of the monitoring data, such as a photoplethysmograms and other physiological parameters.

The invention further provides the technology of a sensor system which has the great advantage that measurement of several physiological parameters can be performed using one single sensor.

As used herein, a "microelectronic system" means a system of electrical connections and/or circuits that facilitate the communication between individual components and the overall functioning of the device. It is to be understood that a microelectronic system have dimensions small enough to make it suitable for incorporation into a device or system that is suitable for attachment to a surface of a subject, such as a human without significantly decreasing the mobility of the subject.

The microelectronic system may comprise one or more application specific integrated circuits (ASIC), electrical system or subsystem, such as, but not limited to, printed circuit boards (PCB), flexible printed circuit boards (FPCB), thick film, thin film, or ceramic technologies or the system or its components may be separately encapsulated.

The microelectronic system of the invention may comprise the following components: Communication components, CPU (central processing unit), power source, storage components, transducer components, interconnections and optionally actuator components.

The CPU (Central Processing Unit) controls and communicates with the components of the microelectronic system. The CPU handles the execution of application software, data decisions making, A/D conversion, DSP (digital signal processing), routing, timing, power management, sleep function, interruption.

The CPU is the component of the microelectronic system controlling other components and optionally making the appropriate data analysis. In general, the more speed and data analysis required, the more power is needed. Therefore a sleep function is often used in order to save power. At certain times or if certain events happen (triggered by a very low power monitoring subsystem) the CPU wakes up, makes the necessary calculations, communicates with relevant components and return to sleep mode. Depending on need very rudimentary CPU to a full-fledged microcontroller can be used according to the invention.

It is to be understood that parts of the data processing unit or a CPU operating specific algorithms may be placed apart from the microelectronic system and may be operating on the basis of data being communicated from microelectronic system.

The term "sensor" as used refers to any component that is capable of detecting any physiological or physical parameter or change of such a parameter in the environment around or nearby the component and which physiological or physical parameter or change of such a parameter optionally through the action of an actuator may by processed in the microelectronic system.

Sensors may include electrical, optical, mechanical, as well as chemical sensors, such as electrodes (polar, bipolar), pressure sensors, needles with electrodes, accelerometers, photo detectors, microphones, ion specific field effect transistors (ISFET), NTC (negative temperature coefficient) resistors, a PTC (negative temperature coefficient) resistor, band gap detectors, ion membranes, enzyme reactors or condensers etc. In particular, the system may comprise non-invasive sensors, e.g. electrodes or optic recognition means. The sensors could, however, also be for invasive capturing of the physiological signals, e.g. in the form of a needle for taking fluid samples, or a needle containing an electrode for subcutaneous capturing of signal.

In addition to the component for capturing of the signal, such as a physiological signal, or as an alternative to the component for capturing of the signal, the interface may comprise an actuator, i.e. a component which converts energy from one form, typically electrical energy, to another body sensible form, which can act on the body of the individual. Examples of such actuator components are electrodes, e.g. for neural- or neuro-stimulation, pumps, injection needles, light emission diodes (LED) or other emitters of electromagnetic radiation, pressure wave generators such as loudspeakers, current generators, or chemical synthesizers.

A "signal" refers to the measuring or detection of any physiological or physical parameter or change of such a parameter by the sensor. A "physiological signal" thus refers to measuring or detection of a physiological parameter or change of such a parameter by the sensor.

"Monitoring data" as used herein refers to a physiological or physical signal that has been transformed to a data signal, which may be processed by a microelectronic system.

To communicate the processed data signal e.g. with an external computer system, with an alarm central or similar surveillance or monitoring system, the device may comprise wireless communication abilities of well known kind. This may include Radio Frequency Identification (RFID) tags which are commercially available in various sizes, ranges and functionality. When the RFID reader applies the appropriate field (e.g. an inductive field) the basic RFID tag return a bit sequence. The sequence is programmed prior to use. RFID range varies from 1 cm to app. 2 meter for passive tags (no power source included) to over 100 meters for active tags (power source included). More sophisticated RFID tags available have storage components where data can be read or stored.

The wireless communication may form part of the microelectronic system, or optionally, it may form part of the interface. As an example, the microelectronic system or the interface may include an RF chip and a coil. Suitable forms of the RFID tag is a RFID tag encapsulated in a glass housing, a RFID tag encapsulated in plastic/epoxy (typically pill shaped), a flat RFID tag with coil and a RF chip laminated between 2 polyimide layers, or a flat RFID tag with large coil antenna with few turns printed on or in the adhesive body and with the RF chip interconnected to the antenna without any further protection/encapsulation.

The wireless communication, in particular in form of a RFID tag, may, when forming part of the interface, be used to identify either the individual, or the type of interface towards the processor. As an example, the identification may relate to the type of signal to which the interface pertains, it may relate to the age of the interface or the duration where the interface was attached to the skin of the individual, the identity of the individual or other characteristics. In some embodiments, the identification tag is embedded in an adhesive foil.

The communication between the device and other devices may be coordinated in a reduced functionality device (RFD) device, e.g. forming part of the microelectronic system. The FFD devices may function at any topology and be the coordinator of the Network, or it may be a coordinator that can talk to any other device. A RFD device is limited to star topology, it cannot become a network coordinator, it talks only to a network coordinator and has very simple implementation. RFDs may be a dedicated network coordinator acting as communication Hub, gateway or router within the Body Area Network (BAN) and handling communication with external unit(s). A communication Hub or gateway may have large storage capacity and store data from the sensor network, and when in proximity with external unit or when otherwise appropriate wirelessly transmit these data.

In particular for monitoring behavior of the individual, or for making combinations between physical activity and other signals, the device may comprise a GPS element, e.g. embedded in the electronic circuit. The system may e.g. log data related to the position, speed or acceleration of the individual or the limp to which the device is attached.

In some embodiments the system according to the invention forms part of a patch with a three-dimensional adhesive body as described in WO 2006/094513, which content is hereby incorporated by reference in its entirety.

The term "three-dimensional" used herein refers to an element e.g. an adhesive body or device or system, having a considerable varying contour when seen in cross section. Thus, for example a three-dimensional adhesive body will have a maximum thickness and a minimal thickness. In some embodiments according to the invention the maximum thickness will be at least twice the thickness of the minimal thickness. In a preferred embodiment the outer rim or the peripheral edge of the adhesive device has a thickness which is less than half of the thickest part of the sensor, normally the central part.

The outer rim of the adhesive body may suitably be shaped circular or oval, with or without flaps and lobes, or it may be shaped rectangular or triangular to obtain as convenient and safe a device as possible.

The pressure sensitive adhesive making up the three-dimensional adhesive body is suitably a mouldable thermoplastic or chemically curing pressure sensitive adhesive having a flexibility enabling the adhesive device to conform to the curvature of body parts while retaining its adhesive properties even under movements.

Suitable, pressure sensitive adhesives making up the adhesive body is an adhesive based on polymers selected from block-copolymers such as styrene-block-copolymers, and hydrogenated styrene-block-copolymers, amorphous poly-alpha-olefins (APAO), polyacrylics, polyvinyl ethers, polyurethanes, polyelhylenevinylacetate, silicone or from the group of hydrogel pressure sensitive adhesives.

Pressure sensitive adhesives based on these polymers are known and the skilled person knows how to prepare adhesives based on these polymers.

Electromyography (EMG) refers to the detection of muscle activity. By electromyography the signal detected be the sensor (or the electromyography) represents the electrical potential generated by muscle cells when these cells are both mechanically active and at rest. The signals from muscle activity may be detected and analyzed in order to detect medical abnormalities or to analyze the biomechanics of human or animal movement.

Galvanic skin response (GSR) also known as electrodermal response (EDR), psychogalvanic reflex (PGR), or skin conductance response (SCR), is a method of measuring the electrical resistance of the skin. The GSR signal is sensitive to emotions in a subject and may be used for the detection and measuring of emotions, such as fear, anger, startle response, orienting response and sexual feelings. Also GSR signals may be used as a lie detector.

Ion specific field effect transistor (ISFET) as used herein refers to a sensor used to measure a particular ion concentration in solution, such as in the interstitial fluid or on the surface of the subject. The gate electrodes of the ISFET sensor are sensitive to certain ions in an electrolyte, so that the gain of the transistor depends on the concentration of these ions.

A thermistor as used herein refers to a resistor whose resistance varies with temperature. The thermistor may be used to measure skin or environmental temperature of the subject wearing the system according to the invention. A negative temperature coefficient (NTC) resistor refers to a sensor wherein the thermal conductivity of a material of the sensor rises with increasing temperature.

Photoplethysmography (PPG) refers to an optically volumetric measurement of an organ, wherein a change in volume, such as one caused by the pressure pulse is detected by illuminating the organ, such as the skin with the light of a light source, such as from a Light Emitting Diode (LED) and then measuring the amount of light either transmitted or reflected to a photodiode. In some preferred embodiments, the photoplethysmography measurement is based on a light reflection.

Arterial oxygen saturation by pulse oximetry ($SpO_2$) refers to the non-invasive measure of the oxygen saturation of a subject's blood by application of photoplethysmography.

Saturation of carbon monoxide (SpCO) refers to the non-invasive measure of carbon monoxide in the blood of a subject by application of photoplethysmography.

Electrocardiography (ECG) refers to a non-invasive recording of the electrical activity of the heart over time. A sensor for measuring ECG refers to the sensors of the electrocardiographic device known to the person skilled in the art.

Electroencephalography (EEG) refers to a non-invasive recording along the scalp of the electrical activity of the neurons within the brain. A sensor for measuring EEG refers to the sensors of the electroencephalographic device known to the person skilled in the art.

Phonocardiogram (PCG) refers to a sound recording of the sounds and murmurs made by the heart. A sensor for measuring a PCG refers to the sensors of the microphones of a phonocardiograph.

It is to be understood that when a photoplethysmographic sensor in the monitoring system according to the present invention is applied at the sternum, the respiration rate is seen very clearly. This enables the monitoring of at least three vital parameters by the same sensor in a wearable device i.e. the heart rate, oxygen saturation, and respiration frequency.

The sternum PPG is an optical signal reflecting the blood flow and pressure. The flow can be interpreted as a flow impacted by two independent pumps. One pump relates to the pulmonary system and the other pump relates to the cardiac system. The separation problem is related to separating the flow caused by the pulmonary pump from the flow caused by the cardiac pump. The respiratory rate (RR) is under most physiological conditions significantly lower than the heart rate. The heart rate is for most parts above 40 beats per minutes. In a clinical setting it would be realistic to set the limits for the RR to be from 5 to 40 per minute. Measurements of RR outside the range of 5 to 40 per minutes should trigger an alarm and not try to estimate the rate further.

One aspect of the invention is estimation of the respiration rate from photoplethysmograms (PPG) measured at the thorax using an optical sensor. The sensor comprises a light sources such as a light emitting diodes (LEDs), a photo detector such a photodiode, and electronic control circuitry such as a amplifiers, converters etc. e.g. combined in a microelectronic application specific integrated circuit (ASIC).

An advantage by placing the patch on the sternum is that this location is very resistant to a decline in perfusion because of the central location on the torso. This is especially valuable during hypothermia and peripheral contraction of the vessels which is seen during conditions such as sepsis and hypovolaemia.

The monitoring system according to the present invention may comprise one or more of the following embodiments:

Photodiodes:

i) High quantum efficiency in the range 390 nm to 1100 nm.

ii) Low capacitance per area i.e. max 1nF/cm2

iii) Surface mountable devices

iv) The photodiodes size should fit to a circle with a radius of 4 mm to 6 mm from the center to the first edge of the photodiodes

v) The photodiodes should preferably have an antireflection coating matched to the refractive index of the gel.

Light emitting diodes:

i) To or more wavelengths in the range 390 nm to 1100 nm, preferably 660 nm and 940 nm

ii) Low optical noise

iii) Surface mountable devices

iv) Small form factor approx. 1 mm by 2 mm

Gels:

i) Transparent, e.g. 50% or more of the light with wavelengths in the range 390 nm to 1100 nm is transmitted per mm gel.

ii) Refractive index of in the range of 1.01 to 1.7 (The refractive index of in vivo tissue is in the range 1.34-1.42 is as disclosed in Tearney, G. J. et al. "Determination of the refractive index of highly scattering human tissue by optical coherence tomography", Opt Lett, 1995, 20, 2258 and Ding, H. et al. "Refractive indices of human skin tissues at eight wavelengths and estimated dispersion relations between 300 and 1600 nm." Phys Med Biol, vol. 51, no. 6, pp. 1479-1489, Mar 2006)

iii) Non-conducting gel; if the gel is in contact with conducting parts of the printed circuit board.

iv) Conduction gel if used for electrical contact to the skin.

Amplifier:

If a general transimpedance amplifier is used it may have the following specifications:

i) The bandwidth should preferable be compatible with simultaneous measurements of a 120 Hz sinusoidal oscillating background light, red PPG, and infrared PPG. E.g. if the signals should be sampled within a maximum of 1% change of the background light normalized with respect to the maximum they should be sampled within 26 $\mu$s. It is possible to have a shorter bandwidth if the sampling frequency is higher than 240 Hz (Nyquist criterion). The background light signal can then be interpolated. The bandwidth should further be compatible with a desired rise time for the photodiodes and amplifier circuit. The rise time represents excess power consumption by the LEDs. E.g. the sampling time of the MSP430 is 4 $\mu$s. If an excess power consumption of the LEDs due to the rise time is 1% then the rise time should be 40 ns, equivalent to a bandwidth of the amplifier of 8.75 MHz. The CC2430 has a sampling frequency of 160 $\mu$s, applying the same requirement gives a bandwidth of 218 kHz.

ii) The operational amplifier should have a low noise. In particular the flicker noise should be low since the flicker noise is likely to be in the same band as the PPG signal.

iii) The gain/noise ratio should be as high as possible and likely higher than $10^9$.

Alternatively a switched integrated transimpedance amplifier can be use to reduce noise by integrating the signal over a time window.

The system according to the present invention may comprise a base suitable for attachment to the surface of the subject. The base may be made from a flexible tape or patch with an adhesive on at least the lower surface which is to face towards the subject and which is therefore intended to bond the device to the subject.

The base may comprise a gel, e.g. a hydrogel with adhesive properties. The hydrogel may or may not be electrically conductive. Different forms or formulations of the hydrogel with different properties may be used within the same system or device, such as a formulation with conductive properties at one place on the base and a formulation with non-conductive properties at another place on the base. The adhesive may form a transmission passage for the physiological signal from the individual to the detecting component. In particular, the passage may be a non-interrupted passage from the place of contact with the individual, e.g. the surface of the skin, to the detecting component. Examples of suitable hydrogels may be obtained from Axelgaard Manufacturing Co., Ltd: http://www.axelgaard.com/home.htm or its subdivision AmGel Technologies; http://www.amgel.com/index.html.

In case of detection of e.g. optic or acoustic physiologic signals, such a non-interrupted passage in one and the same material, namely the adhesive (such as a gel), provides for a minimal loss of signal strength and quality, such as by preventing reflection, scattering, and refraction in an interface between materials with different properties such as refractive indices.

The base may comprise an adhesive or gel which amends the physiological signal, e.g. a gel which modifies an optical signal, filters an electrical signal or dampens an acoustic signal.

In particular, it may be an advantage to use an adhesive, e.g. in form of a hydrogel or similar soft solid material, which is adhesive, adaptable to human skin, conductive or non-conductive, transparent or non-transparent and for optical sensors non-scattering a with a viscosity or flexibility in a suitable range, and it may further be an advantage to use a material with a refractive index in the range of 1.01-1.7, e.g. 1.30-1.45, such as 1.34-1.42. In this way, the index becomes close to that of average skin whereby reflection of the physiological signal, be that an acoustic or optic signal, can be prevented or at least reduced.

Discrete Saturation Transform (DST®) algorithm refers to a mathematical method used to calculate $SpO_2$ in pulse oximetri. The method is developed by Masimo Corporation. The DST algorithm allows one to separate and, consequently, calculate the optical density ratios that correspond to both the arterial oxygen saturation ($r_a$) and an estimate of the venous oxygen saturation ($r_v$).

Independent Component Analysis (ICA) algorithm refers to the computational method for separating a multivariate signal into additive subcomponents supposing the mutual statistical independence of the non-Gaussian source signals. Sensors and ICA may be as described in WO 03039340, US 6701170, US 7079880, and/or US 7343187 the content of which is hereby incorporated by reference in its entirety.

In some important aspects the monitoring system according to the present invention measures one or more vital parameter. As used herein the term "vital parameter" refers to a physiological parameter where total failure will lead to death of the organism. Among the vital physiological functions is the respiratory function and hence the respiratory rate is a vital parameter and pivotal for the clinical observation of patients. Respiration rate is affected in many conditions such as hypercapnia, hypoxia, stress, fever, pain, sleep apnoea, chronic obstructive pulmonary disease, sudden infant death syndrome, postoperative and central nervous system depression. Finally, importance of the respiration rate is reflected by being one of the physiological parameters, which can trigger the activation of The Medical Emergency Team in many hospitals.

Accordingly, in some embodiments the system according to the present invention is configured to communicate with another device, such as a mobile phone or central monitoring system of a hospital. The system according to the present invention may be configured to communicate with of the patient, a clinician, a spouse, a family member, a caregiver, or a medical provider, when the values received from the first and/or second sensor are within specific physiological ranges. This may allow for therapeutic intervention to prevent a critical condition, such as death, when the values received from the first and/or second sensor are not within acceptable physiological ranges.

In some embodiments the monitoring system according to the present invention is a wireless monitoring patch which can measure the respiration rate, heart rate, and oxygen saturation by sensors integrated and embedded in the patch. The monitoring system in this context may improve the patient comfort, and in addition it may enable patients to be mobile and not constrained to a specific location e.g. a bed.

In some embodiments the monitoring system according to the present invention provides a convenient and improved method to monitor the respiration and other physiological parameters under the circumstances experienced in a hospital setting.

In some embodiments the monitoring system according to the present invention may monitor respiration on a single spot on the body without the use of tubes for airflow, additional wires, or additional electrodes. For example, the invention solves the problem, wherein patients undergoing surgery is monitored by wired devices and apparatuses with may be disconnected and prevent easy access to the patient under surgery. Thus, the invention improves the monitoring of the patient during anaesthesia and transportation of the patient in the hospital facility where wired systems are difficult to handle due to the wired connection between the patient and the monitoring equipment.

In some embodiments the monitoring system according to the present invention measures the optical PPG signals at the sternum by the use of an annular photo detector where the light sources are placed in the middle of the surrounding photoactive area in a distance of 4 - 7 mm away from the light source. One such suitable photo detector is disclosed by Duun et al. Jour. Micromech. Microeng. 20 (2010).

In some embodiments the monitoring system according to the present invention is a wearable and wireless system with a 3-dimensional adhesive device wherein the optical sensor is embedded along with power source, wireless communication, and electronics. A suitable 3-dimensional adhesive device where sensors and microelectronic may be embedded is disclosed in WO 2006/094513.

As described elsewhere the present invention provides in some embodiment an intelligent or adaptive monitoring system or device, which system may provide an output of data limited to the most critical and essential physiological parameters of the subject and with the lowest requirement or consumption of resources, such as a resource selected from time, power, power management, power source, power size, data size/information size, prize/socioeconomic cost, comfort/discomfort to the subject wearing the system, side effects, processing power, data storage, consumable, lifetime, connectivity/availability, such as with external resources/internet, and environmental load.

The term "control" as used herein means that sensor 2 is configured to turn on/off, or change some predefined settings in response to a signal from the first sensor. In terms of the present invention, the "control" may be "intelligent" or "adaptive", meaning that the system of sensors may be configured to work in an optimal setting with respect to one or more parameter or resource requirement as mentioned above. The signal from sensor 1 may be in response of the first signal being turned on/off, in response to a difference in a value of one or more parameter measured at two different time points.

The microelectronic system according to the present invention may be capable of identifying a control method while running and under consideration of a sensed parameter.

Accordingly, in some embodiments, in the system according to the present invention the control method defines at least one of:

  i) a resource optimization.

  ii) a sensor selection between the first and second sensor.

  iii) a sampling frequency of data requisition from the sensors.

  iv) a configuration of data processing of data received from the first and second sensors.

Accordingly the monitoring system according to the present invention may be optimized with respect to a resource which is not related only to power consumption. Thus, in some embodiments the energy consumption is substantially unchanged when a first signal trigger the measurement of a second signal, such as a more critical and essential physiological signal. In some embodiments the first signal trigger the measurement of a second signal, which second signal is more precise or contain more information, such as in combination with the signal obtained from the first signal. In some embodiments the second sensor triggered by the signal of the first sensor is not simply configured to verify or repeat the signal obtained from the first sensor to confirm a physiological status in the subject.

In some specific embodiments the system is streaming continuously data to a data processing unit based on a signal from the first sensor.

In some embodiments the first and second signal is obtained from same sensor or same type of sensor. Also the first and second signal may be essentially the same (obtained from same or different sensors), such as the same physiological signal with different quality or precision. This may be a measure of the ECG with a small resolution (e.g. 8 bit) or a pulse detector to establish a pulse and when certain events occurs, such as a specific physiological state of the subject, a second sensor is triggered to obtain a more precise ECG (24 bit A/D). Also ECG or EMG can be measured continuously with a simple low power consuming front end, like detecting certain shift in pulse or muscle activity, when these shifts occur the corresponding signal is obtained in good or better resolution. For ECG and EMG the signals can be measured with low sample rate and with certain conditions occur it can measure with high sample rate.

In other embodiments the monitoring system according to the present invention measures simple pulse or skin contact with a first sensor and only in the event of a positive output from the first sensor, when it is evaluated that the system is placed on a human, the system will trigger sensor 2.

In other embodiments the monitoring system according to the present invention has a first sensor measuring electrical signals with electrodes, such as a measuring of ECG or respiration rate. Under certain conditions (to be programmed in the data processing unit) the signal from such first sensor will trigger a second sensor that can measure a second optical signal, such as to measure the respiration rate optically.

In other embodiments the monitoring system according to the present invention has a first sensor measuring the skin temperature (e.g. of a fireman) or another non-physiological temperature. When the temperature exceeds a certain level (to be programmed in the data processing unit) that is deemed hazardous for the fireman the systems triggers a second sensor or a multitude of sensors like ECG, oxygen saturation.

*Specific embodiments of the invention*

As described above the present invention relates to a minimal-invasive monitoring system suitable for attachment to a surface of a subject, the system comprising at least one first sensor which can receive a first signal and at least one second sensor which can receive a second physiological signal from the subject, the sensors being controlled by a microelectronic system being wearable by the subject, powered by independent powering, and comprising a communication structure optionally for wireless transfer of monitoring data, wherein the monitoring data based on the second physiological signal is under control of the monitoring data based on the first signal.

As used herein minimal-invasive refers to a device or system, which is functioning essentially on the surface of a subject, such as non-invasively without in any way penetrating the surface of the subject. In most applications the sensors of the system is receiving signals through the skin of the subject, such as with electrodes of electrocardiography (ECG) sensor. In some applications however, the sensor may have minor electrodes, such as gate electrodes of an ISFET sensor, penetrating the skin of the subject. In other applications, the sensor may in other ways amend the characteristics of the skin, e.g. by etching, heating, radiation, e.g. by microwaves or ultrasound. As used herein minimal-invasive therefore refers not only to non-invasive but also to invasive systems e.g. of the mentioned kind.

In some embodiments the system according to the present invention is contained within a single device.

In some embodiments the system according to the present invention comprises independent means capable of providing electrical power for the microelectronic system for a period of time at least sufficient to capture the physiological signal from the subject.

In some embodiments the system according to the present invention is non-invasive.

In some embodiments in the system according to the present invention the first signal is a physiological signal from the subject.

In some embodiments in the system according to the present invention the physiological signal or said monitoring data based on the first signal is one or more selected from heart rate (HR), respiration, such as respiration rate, skin and/or core body temperature, snoring sound or other sounds of the subject, electromyography (EMG), such as submental EMG, galvanic skin response (GSR), electrocardiography (ECG), electroencephalography (EEG), phonocardiogram (PCG), arterial oxygen saturation ($SpO_2$), muscle activity, motion, emotions, arterial saturation of carbon monoxide (SpCO), sensors for physiological gases, such as a gas exhaled from the lungs, such as exhaled nitrogen oxide.

As used herein "motion" refers to any change in the location of a body or body part. Accordingly "motion" may include but is not limited to movement of a subject from one place to another, movement of various external body parts, such a movement of body extremities, chills, spasms, involuntary body movements associated with seizures and the like. In some embodiments in the system according to the present invention the first signal is a non-physiological signal.

In some embodiments in the system according to the present invention the non-physiological signal is obtained from one or more selected from a Global Positioning System (GPS), a pressure sensor, an accelerometer, air humidity, environment temperature, predetermined and specific radio signal or lack of the same, Radio Frequency Identification (RFID) tag, chemical or biochemical sensors, such as for toxic or hazardous gases, on-demand signal from the subject or another person responsible for monitoring the physiological signal from the subject.

As used herein radio signal refers to any transmission of electromagnetic waves with a frequency suitable for transmission through the air or the vacuum of space, such as frequencies below those of visible light. The radio signal may be location specific. It is to be understood that the system according to the present invention may be under influence of a constant radio signal, which is turned of under specific conditions, such as when the system is placed in a specific location. Accordingly, the signal may be when the radio signal is turned off. Alternatively, a signal may received when a radio signal is turned on, such as when the system is placed in a location, where the radio signal is active and received by the system.

In some embodiments the system according to the present invention is part of a patch with a three-dimensional adhesive body.

In some embodiments the system according to the present invention further comprises a disposable part containing an adhesive material.

In some embodiments in the system according to the present invention a disposable part provides for energy, such as an exchangeable battery or a fuel cell.

In some embodiments in the system according to the present invention the low power electronics comprises components selected from communication component, Central Processing Unit (CPU), strain gauge, storage component, transducer component, actuator component and electrical interconnections between the components.

In some embodiments in the system according to the present invention the transducer has a detecting element selected from electrodes (polar, bipolar), a pressure sensor, an accelerometer, a photo detector, a microphone, ion specific field effect transistors (ISFET), thermistor, such as a negative temperature coefficient (NTC) resistor, a band gab detector, an ion membrane, an enzyme detector or a condenser.

In some embodiments in the system according to the present invention the microelectronic system comprises a Network HUB, gateway, or network coordinator.

In some embodiments in the system according to the present invention the microelectronic system includes a Global Positioning System (GPS).

In some embodiments in the system according to the present invention the microelectronic system includes a Radio Frequency Identification (RFID) tag.

In some embodiments in the system according to the present invention the first and/or second sensor is for the optical measurement based on photoplethysmography (PPG).

In some embodiments in the system according to the present invention the first and/or second sensor is for optical measurements of one or more physiological signal selected from respiration, such as respiration frequency, heart function, heart rate (HR), arterial oxygen saturation by pulse oximetry ($SpO_2$), saturation of carbon monoxide (SpCO), methaemoglonin (metHb), blood pressure, perfusion index, parameters associated with heart rate like e.g. heart rate variability, tissue perfusion, and haemoglobin concentration.

In some embodiments in the system according to the present invention the first and/or second sensor is for measuring electric potentials.

In some embodiments in the system according to the present invention the first and/or second sensor is for measuring one or more physiological signal selected from electrocardiography (ECG), electromyography (EMG) electroencephalography (EEG), galvanic skin response (GSR), phonocardiogram (PCG), arterial oxygen saturation ($SpO_2$), muscle activity, emotions, arterial saturation of carbon monoxide (SpCO), blood carbon dioxide ($CO_2$) and different forms thereof, blood pH, blood pressure (BP), blood pH, respiration, such as respiration frequency (RF), heart function, heart rate (HR), bioimpedance, and/or rhythm, heart sounds, respiratory sounds, blood pressure, posture, wake/sleep, orthopnea, heat flux, patient activity, snoring sound or other sounds of the subject, and temperature, such as skin temperature (ST), and/or core body temperature.

In some embodiments in the system according to the present invention the first and/or second sensor is for mechanical measurements for measuring one or more physiological parameter selected from respiration, such as respiration frequency, blood pressure, sweat production, tissue perfusion, function of heart, including its valves and vessels, and motion.

In some embodiments in the system according to the present invention the mechanical measurements is selected from ultrasound based measurements and/or a phonocardiogram (PCG).

In some embodiments the system according to the present invention has an average diameter of less than about 100 mm.

In some embodiments the system according to the present invention has a thickness of less than about 10 mm, such as less than about 9 mm, such as less than about 8 mm, such as less than about 7 mm, such as less than about 6 mm, such as less than about 5 mm.

In some embodiments the system according to the present invention is suitable for attachment and application on sternum of a human being.

In some embodiments in the system according to the present invention the first and/or second sensor is a sensor for motion detection.

In some embodiments the system according to the present invention is suitable for indicating convulsions during sleep, cardiovascular disorders including heart disorders and cardiac arrhythmias, tachycardia, hypertension, hypotension, chronic obstructive lung disease (COLD), sleep apnea, vital life signs, pain relief treatment such as with morphine, seizures, such as epileptic seizures, muscle spasms, burns, hypoxia, acidemia, hyper- and hypo-glycemia, hypothermia, and hyperthermia.

In some embodiments in the system according to the present invention at least two physiological signals from the subject are monitored.

In some embodiments the system according to the present invention is streaming continuously data to a data processing unit based on a signal from at least one sensor.

In some embodiments the system according to the present invention is concentrating data to send data to a data processing unit in a data package.

In some embodiments in the system according to the present invention the first signal and the second signal are different.

In some embodiments the system comprises at least one light source and at least on photodetector.

In some embodiments in the system the lightsource is LED or LEDs.

In some embodiments in the system the photodetector is a single ring shaped photodiode with the lightsource(s) in the middle.

In some embodiments in the system the photodetector is multiple photodiodes placed around the lightsource(s) in the middle.

In some embodiments in the system according to the present invention, the second physiological signal is different from the signal obtained from first sensor.

In some aspects of the invention relates to a system comprising a monitoring system, and a data processing unit receiving monitoring data from the monitoring system and operating an algorithm based on the monitoring data from the first and/or second sensor to provide an output that control the monitoring data of the second sensor, or an output indicating the state of at least one physiological parameter of a subject carrying the monitoring system. Accordingly the data processing unit in the system according to the present invention may be configured to turn on and off the first and/or second sensor, such as based on the signal from the first sensor. The data processing unit in the system according to the present invention may also be configured to communicate an output from the first and/or second sensor, such as by the streaming of data or simply by the triggering of an alarm.

The phrase "under control of the monitoring data based on the first signal" as used refers to the system according to the present invention wherein the data processing unit in the system is configured so that a second sensor is turned on or of depending on the processing of the monitoring data based on the first signal taking into consideration requirements or resources, selected from time, power, power management, power source, power size, data size/information size, prize/socioeconomic cost, comfort/discomfort to the subject wearing the system, side effects, processing power, data storage, consumable, lifetime, connectivity/availability, such as with external resources/internet, and environmental load.

In some embodiments according to the present invention, in this system the algorithm is independently selected from a Discrete Saturation Transform (DST) or an Independent Component Analysis (ICA).

In some embodiments according to the present invention, in this system the output controls the monitoring data of the second sensor to provide another monitoring signal from the first sensor.

It is to be understood that in some situations it may be advantages to have confirmed the monitoring of a physiological signal. Accordingly, in some embodiments the output of a sensor triggers or controls the repetition of the monitoring of a physiological signal. In the event of a first sensor receiving a physiological signal, the monitoring of a second physiological signal may just repeat the monitoring of the first signal. In the event of a first sensor receiving a non-physiological signal, this signal will control the monitoring of a second physiological signal, which in turn may control the sensor of the second physiological signal to repeat the monitoring signal.

In some aspects the present invention relates to a method for monitoring at least one physiological parameter of a subject, wherein a monitoring system according to present invention is placed on the surface of a subject and data from the system according to the invention provide an output indicating the state of at least one physiological parameter of a subject carrying the monitoring system.

In some embodiments according to the present invention, the physiological parameter or representation of a physiological parameter of a subject is selected from body temperature, blood pH, blood pressure, respiration, such as respiration frequency, heart function, heart rate (HR), arterial oxygen saturation ($SpO_2$), saturation of carbon monoxide (SpCO), electrocardiogram (ECG), electromyogram (EMG), electroencephalogram (EEG), skin temperature, emotions, sweat production, tissue perfusion, function of heart, including its valves and vessels, motion, methaemoglonin (metHb), heart rate variability, tissue perfusion, and haemoglobin concentration.

In some embodiments according to the present invention the state of at least one physiological parameter of a subject carrying the monitoring system is independently selected from convulsions during sleep, cardiovascular disorders including heart disorders and cardiac arrhythmias, tachycardia, hypertension, hypotension, chronic obstructive lung disease (COLD), sleep apnea, vital life signs, pain relief treatment such as with morphine, seizures, hypoxia, acidemia, hyper- and hypo-glycemia, hypothermia, and hyperthermia.

In some embodiments according to the present invention the physiological parameter is measured during work, such as during the work of fire fighters or military personnel.

In some embodiments according to the present invention the physiological parameter is measured on a hospitalized subject or alternatively on a disease subject staying at home.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

All methods described herein can be performed in any suitable order unless otherwise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a formulation described herein as comprising a particular element should be understood as also describing a formulation consisting of that element, unless otherwise stated or clearly contradicted by context).

This invention includes all modifications and equivalents of the subject matter recited in the aspects or claims presented herein to the maximum extent permitted by applicable law.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in micro electronic systems, medical devices, or related fields are intended to be within the scope of the following claims.

EXAMPLE 1

The following table provides some exemplary embodiments for the combination of the first and second sensor.

| Second Physiclogical signal First signal | HR | RF | SpO$_2$ | SpCO | ECG | EMG | EEG | GSR | PCG | BP | ST |
|---|---|---|---|---|---|---|---|---|---|---|---|
| HR | X | X | X | X | X | X | X | X | X | X | X |
| Respiration rate | X | X | X | X | X | X | X | X | X | X | X |
| Skin and/or body temperature | X | X | X | X | X | X | X | X | X | X | X |
| Snoring sound | X | X | X | X | X | X | X | X | X | X | X |
| EMG | X | X | X | X | X | X | X | X | X | X | X |
| GSR | X | X | X | X | X | X | X | X | X | X | X |
| ECG | X | X | X | X | X | X | X | X | X | X | X |
| EEG | X | X | X | X | X | X | X | X | X | X | X |
| PCG | X | X | X | X | X | X | X | X | X | X | X |
| SpO$_2$ | X | X | X | X | X | X | X | X | X | X | X |
| Muscle activity | X | X | X | X | X | X | X | X | X | X | X |
| emotions | X | X | X | X | X | X | X | X | X | X | X |
| SpCO | X | X | X | X | X | X | X | X | X | X | X |
| GPS | X | X | X | X | X | X | X | X | X | X | X |
| Pressure sensor | X | X | X | X | X | X | X | X | X | X | X |
| Accelerometer | X | X | X | X | X | X | X | X | X | X | X |
| Air humidity | X | X | X | X | X | X | X | X | X | X | X |
| Environment temperature | X | X | X | X | X | X | X | X | X | X | X |
| Specific radio signal | X | X | X | X | X | X | X | X | X | X | X |

EXAMPLE 2

Monitoring patch for monitoring of EMG and $SpO_2$ by pulse oximetry.

Based on the following considerations a wireless health system was developed as an electronic patch. The Electronic Patch is a genuine platform which is compatible with many types of sensors. The patch according to this example describe two applications: monitoring of EMG and $SpO_2$ by pulse oximetry. The EMG sensor is intended for detection of convulsions during sleep and the pulse oximetry sensor is intended for people suffering from heart disorders, chronic obstructive lung disease (COLD), sleep apnea, and professionals during work such as fire fighters. The Electronic Patch consists of a printed circuited board (PCB) where sensors are mounted on the bottom, and the top contains all the electronics and radio communication. The PCB is encapsulated in a hard plastic box and attached to the body by an adhesive material of hydrocolloid polymer.

Sensors

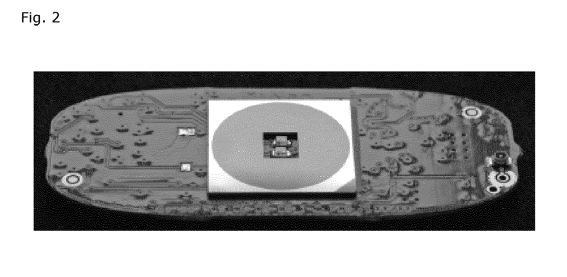

The EMG sensor have a standard design made by three silver electrodes distributed evenly on the PCB with a separation of 10 mm. The pulse oximetry sensor comprises a concentric photodiode with two LEDs in the middle a red (660 nm) and infrared (940 nm). The sensor is shown in Fig. 2.

Electronics

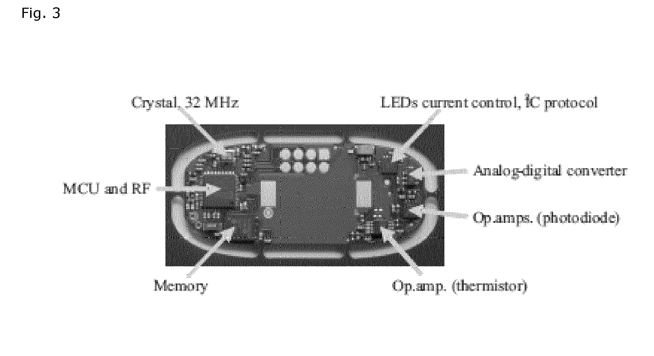

The top side of the PCB contains the electronics as shown in Fig. 3. It contains analog frontend electronics, a low power microprocessor with a built-in radio, and memory. The microprocessor uses from 190 $\mu$A at 32 kHz with the radio off to 27 mA at 32 MHz with the radio on. The power usage of the microprocessor will be application dependent. In the pulse oximetry sensor an I2C current controller to control the LEDs is also present. The patch is powered by a coin size 3 V Lithium-ion battery with 170 mAh.

Wireless communication and network

The wireless networking in the Electronic Patch is based on a 2.4 GHz radio and a proprietary protocol which allows the patch to work in a wireless personal area network, but not as an independent system in direct contact with service providers or hospitals. However, this contact can be made by external access points connected to the internet e.g. smart phones. Access points could also be installed in the person's home or other daily environments. The advantage using this solution is that power consuming long distance communication is placed outside the patch. This configuration also supports the service of many patches. For instance in the case of assisted living homes where many elderly could be monitored by individual patches each connected to the same network of access points covering the entire estate. A proprietary protocol has been employed instead of the ZigBee and Bluetooth protocols due to lower power consumption. The drawback is a limited range of a few meters. This would be increased by using the Bluetooth protocol.

Mechanical assembly

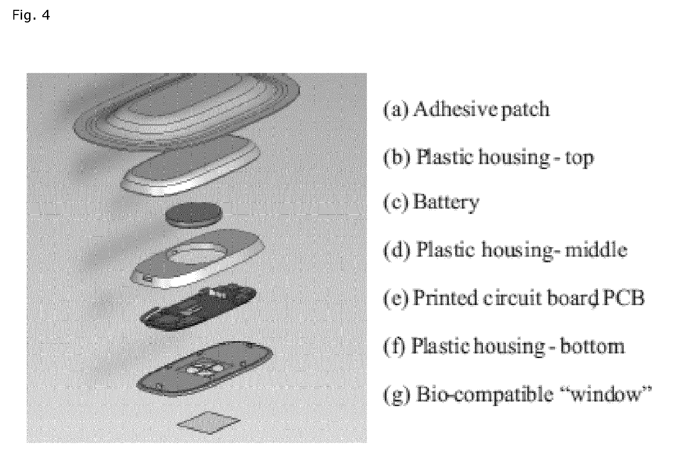

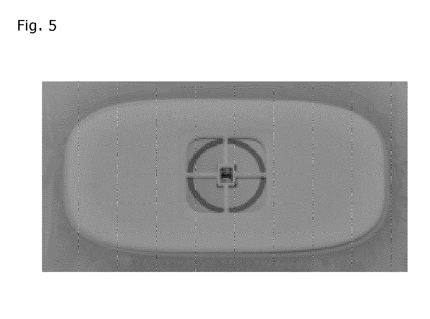

The mechanical assembly is shown in Fig. 4 and the final patch with the pulse oximetry sensor is shown in Fig. 5. Sensors and electronics are encapsulated in a bio-compatible plastic housing which protects the electronics from sweat and moisture. The pulse oximetry sensor is further protected by an epoxy seal with tuned refractive index optimized for maximum transmittance of light and the EMG sensor has an epoxy seal. With this solution the system can even be warn during a shower. The patch comes in two parts: 1) A reusable sensor part consisting of a bottom- (f) and middle plastic housing (d), sensors and electronics (e). 2) A disposable part consisting of the adhesive patch (a), top housing (b), and battery (c). The adhesive patch has to be changed once every week due to dead skin cells. This is therefore the period which the battery has been designed to last. The adhesive patch is designed for attaching the plastic housing onto the skin and the hydrocolloid polymer allows for diffusion of moisture away from the skin.

EMG application

Electromyography is a method of detecting muscle activity. The method relies on the change of membrane potential of the muscle cells with muscle activity. The resting muscle cell has a potential across the cell membrane of approximately -90 mV. During muscle activity the membrane potential change to approximately 15 mV. This can occur both in spikes when the muscle is stimulated or constantly when the muscle contraction is tetanic. EMG can be measured both non-invasively on the skin surface above the muscle or invasively by needles. A standard configuration was used for surface EMG where the potential is measured between two electrodes relative to a third electrode placed in between. The measured signal is amplified, and to save power an analog circuit for detection of spikes has been employed. The microprocessor is then only turned on whenever spikes are detected and the muscle is active. The microprocessor then analyzes the EMG signal and evaluate if convulsions are taking place.

Pulse oximetry application

A pulse oximetry sensor detects pulse and arterial oxygen saturation. It is an optical technique invented by T. Aoyagi in 1972 and is based on absorption changes of light with the blood flow. Pulse oximetry relies on the difference in the absorption spectra between oxygenated haemoglobin ($HbO_2$) and deoxygenated haemoglobin (Hb). It has been shown that the ratio between absorption coefficients of $HbO_2$ and Hb makes wavelengths of 660 nm and 940 nm suitable. For the pulse oximetry application a custom design silicon photodiode may be chosen. This allows for optimization of the photodiodes for the pulse oximetry application. To minimize the necessary driving current of the LEDs a fabricated large area photodiodes which are concentric around the LEDs and hence optimized for collection of backscattered light from the tissue, is used. The photodiodes have a chip size of 14 mm by 14 mm and with various active areas ranging from 22 $mm^2$ to 121 $mm^2$. This area is up to 20 times larger than what is used in a Nellcor wired reflectance sensor. The largest photodiode is shown in Fig. 2. Increasing the photodiode area also increases the capacitance and this will lower the speed of the photodiode, hence there is a trade-off between photodiode area and speed. In this system a sampling rate, fs, of 1 kHz, is used. The capacitances of the largest photodiodes are 24 nF $\pm 2$ nF. Given a photodiode transimpedance amplifier circuit with a $10^4$ amplification the bandwidth, BW, will approximately be given by:

$$BW \approx (C_{PD} \cdot R_{Amp})^{-1} = (24nF \cdot 10k\Omega)^{-1} = 4 \ kHz$$

Several 1 mm wide rings with radii from 3.5 mm to 6.5 mm were fabricated. This is done to gain knowledge about at what radii on a specific body location the signal has the best signal to noise ratio. One such ring sensor is seen in Fig. 5. To ease the assembly it was chosen to make backside photodiodes which have the junction and both contacts on the side facing the PCB. Therefore, no wirebonding is necessary. To shield from ambient light and to optimize transmission at the two wavelengths of interest i.e. 660 nm and 940 nm a two layer antireflection filter consisting of 550 nm PECVD silicon nitride on 50 nm thermal dry silicon oxide has been employed. This filter reach optical transmission > 98% at 660 nm and 940 nm and suppressing other wavelengths to approximately 50 % in the range 600 nm - 1100 nm. For wavelengths below 600 nm the tissue absorption is very strong and hence ambient light at these wavelengths does not course problems. The photodiodes are also patterned with Aluminium on the side of the light entrance to give a well defined area of light gathering. From the PPGs the pulse and the oxygen saturation can be calculated. To further optimize the power consumption of the pulse oximetry sensor the duty cycle of the LEDs, DLED, can be considered. The minimal duty cycle that is possible, when at least 95 % of the LED power must be maintained, is given by the sampling frequency and the bandwidth of the photodiode amplifier circuit. In the present case

$$D_{LED} \approx 2 \cdot fs/BW = 2 \cdot 1kHz/4kHz = 50\%$$

When lit the LEDs typically use 20 mA at 1.5 V. The I2C current controller needs 10 mA at 3 V to deliver 20 mA at 1.5 V. Having a duty cycle of 50% on the LEDs the I2C current controller on average will use 5 mA at 3 V. If measured continuously the LEDs alone would use the battery in 34 hours. Therefore, one would like to reduce the LED power consumption by at least a factor of 10. Because then one can measure continuously for a week and only use 85 mAh or half the battery power available on the LEDs. One way to do this will be to improve the speed of the photodiode amplifier circuit by lowering the photodiode capacitance.

EXAMPLE 3

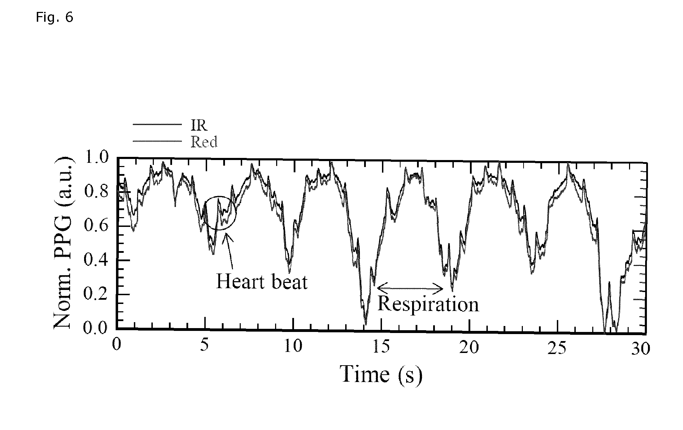

Fig. 6 shows the measured PPG signal when the patch described in Example 1 is mounted on the sternum. The measured signal contains information of both the respiration rate, the heart beat rate, pulse and the oxygen saturation. The respiration rate is very clearly seen and in this case it is found to have a period of 5s corresponding to 12 respiration cycles/minute. Thus, at the sternum position the device can measure the conventional PPG signal and the respiration rate.

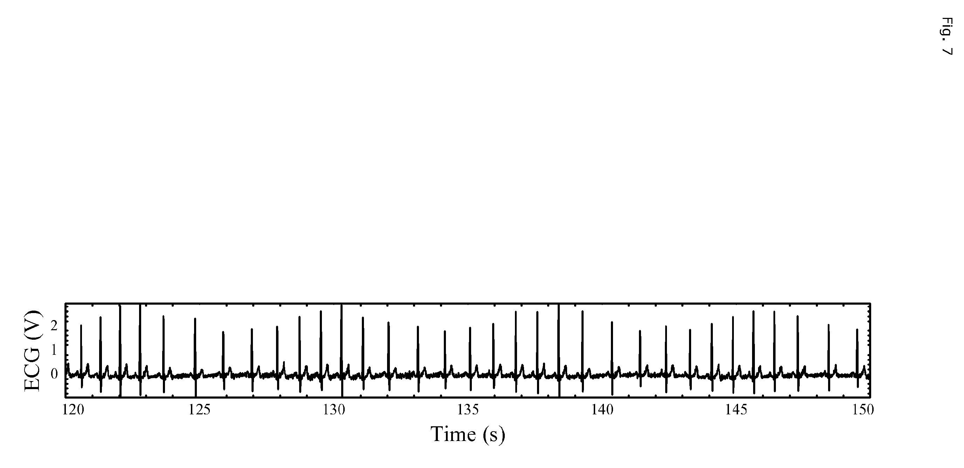

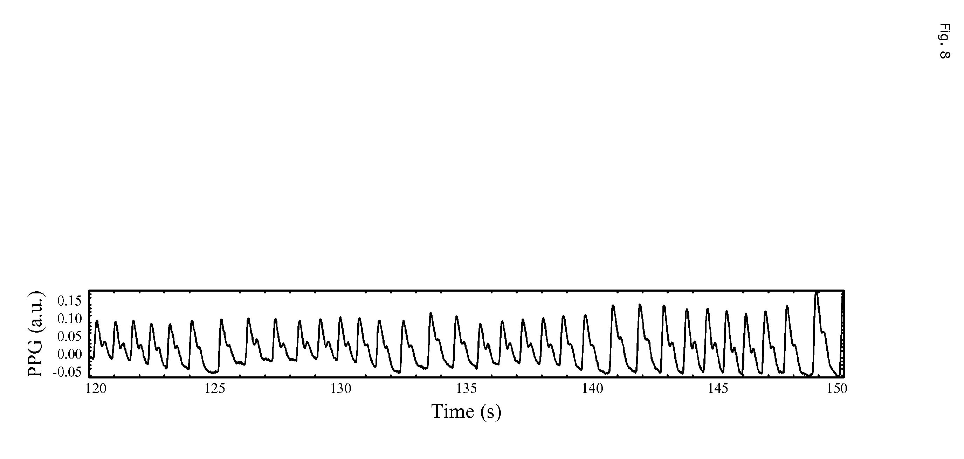

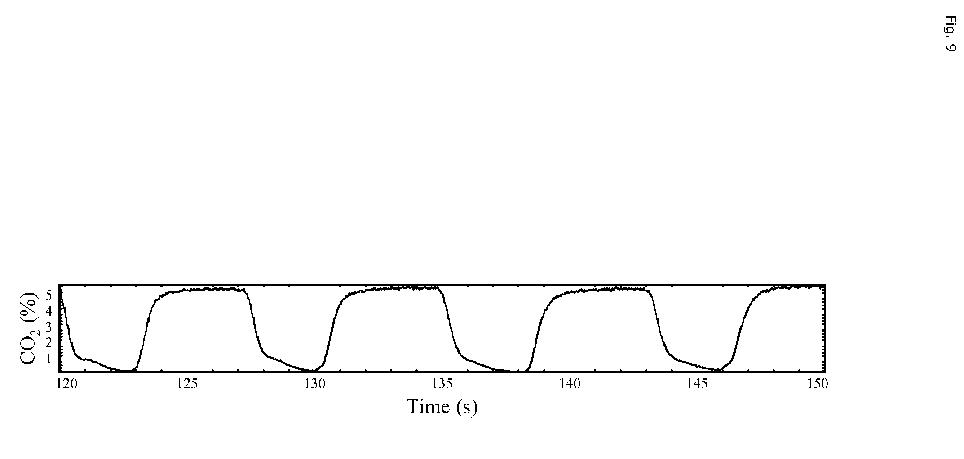

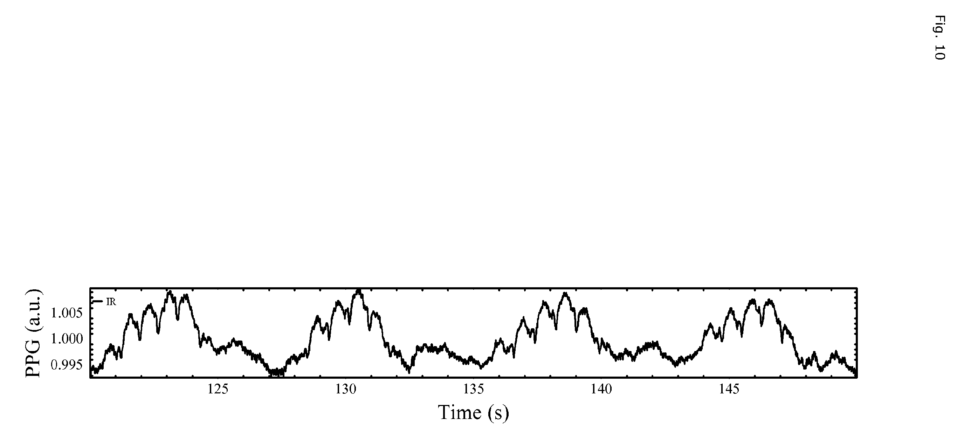

Fig. 7 to 10 show the relationship between the sternum PPG signal, heart rate and respiration rate. The sternum PPG in Fig. 10 has two frequency components: The component with the longer period and relatively larger amplitude relates to the respiration as seen by comparing with Fig. 9 which shows the fraction of $CO_2$ in the airflow. The component with the shorter period relates to the heart rate. This is seen by comparing with Fig. 7 which shows the ECG.

Accordingly, it is illustrated that the monitoring system according to the present invention in addition to the pulse and two PPGs for estimation of the oxygen saturation solves the problem of measuring the respiration rate by a conveniently and non-invasively spot measurement using an optical sensor embedded in a 3-dimensional adhesive patch.

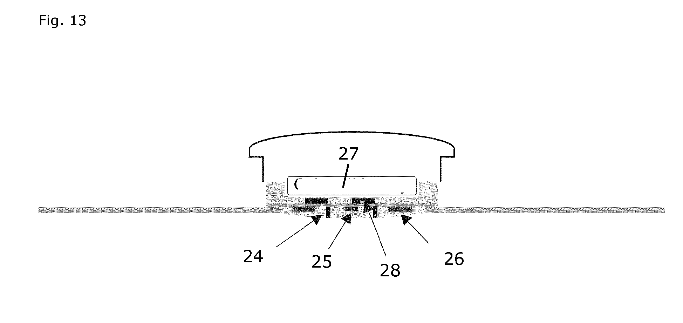

One suitable layout and geometry of optical sensor comprising electro optic components of light emitting diodes (LEDs) and photodiodes is illustrated in fig. 13. The geometry and separation between the LEDs and photodiodes is essential as this influences the quality of measured photoplethysmograms (PPGs). Preferably, the separation between the LEDs and photodiodes should be in the range 4 mm to 7 mm.

Example 4

Device for measuring photoplethysmograms (PPGs), suitable for use in a device according to the present invention:

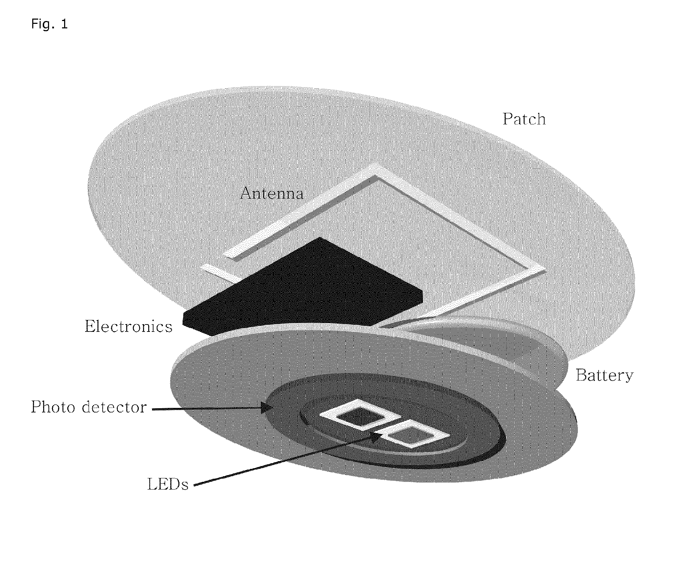

The device has two parts, a reusable and a disposable: The reusable part, the "Sensor Housing", contains the sensors and electronics encapsulated in a plastic housing as seen in the lower part of Fig. 1. The disposable part, the "Adhesive Cap", comprise a Battery Frame and battery embedded in an adhesive patch as seen in the upper part of Fig. 1. The two parts are detachable attachable by snap latches. The sensor house has the dimensions 56 mm x 28 mm and is 4 mm thick at the centre. The adhesive cap has dimensions of 88 mm x 60 mm and is 5 mm thick at the centre. This is also the dimensions of the assembled patch. The weight of the assembled patch is 16 g. The plastic parts (Bottom Housing, Top Housing and Battery Frame) are manufactured in polylaurinlactam (PA12 or Nylon) using Selective Laser Sintering (SLS) a 3D printing. Adhesive (Loctite 4031) is used for assembly of the PCB in the housing and the battery in the battery frame. The adhesive used is a mixture containing a water-swellable hydrocolloid and a water-insoluble, viscous and elastomeric binder. It is 3-dimensionally structured so that it is thicker in the centre relative to the edges.

The sensor comprises two commercial LEDs, at wavelengths of 660 nm (Lumex Inc.) and 940 nm (Stanley Electric Co., Ltd.), placed in the center of an annular backside silicon photodiode. The annular photodiode is used to reduce the current consumption in the LEDs. The photodiode has a defined aperture in a distance of 4-7 mm from the centre. The aperture is made by a deposition of an aluminum layer.

The electronic components, apart from the photodiode, are soldered to the printed circuit board using standard surface mounting technology. The photodiode is mounted using a CW2400 conducting epoxy (Circuitworks) and a Chipcoat 8426 underfiller (Namics) for good mechanical adhesion. The hole for the light emitting diodes (LEDs) and the photodiode in the bottom housing is sealed using an optically transparent epoxy Epo-Tek 302-3M (Epoxy Technology Inc.). The epoxy has a thickness of approximately 300 $\mu$m. The epoxy has a refractive index of 1.56 which is close to the refractive index of the human skin. In human skin the refractive index of the outer skin layer, the epidermis, is in the range 1.34 - 1.43 at wavelengths of 660 nm and 1.42 at 940 nm. The photodiode has an optical filter for antireflection with is matched for the epoxy sealing. Hence, it is matched to the refractive index 1.56 of the epoxy. It is important that the epoxy has an optical thickness greater than the typical coherence length of the LEDs to avoid unwanted interference. The coherence length of an typical LED is 50-100 $\mu$m and the optical thickness of the epoxy layer is approximately 470 $\mu$m. The transmission is better than 90% at wavelengths 660 nm and 940 nm at angles of incidence ranging from 0 to 60 degrees.

**Claims**

1. A minimal-invasive monitoring system configured to be attached to a skin surface in front of the sternum of a subject by means of an adhesive,

    - said monitoring system being contained within a patch, where the adhesive is configured to at least retain its adhesive properties under movement,
    - said monitoring system comprising at least one first sensor, at least one second sensor for the optical measurement based on photoplethysmography (PPG), and a microelectronic system being wearable by the subject, where the at least one first sensor is configured to receive a first signal, where the at least one second sensor is configured to receive a second physiological signal based on photoplethysmography (PPG) from the sternum of said subject, wherein the second physiological signal is different from the first signal and the first sensor is different from the second sensor, the sensors being controlled by said microelectronic system,
    - the microelectronic system being powered by independent powering, the microelectronic system comprising an optional communication structure for transfer of data signals from the first and second sensors,
    - the monitoring system further comprises a data processing unit configured to receive the data signals from said monitoring system, the data processing unit being an integral part of the microelectronic system, and where the data processing unit is configured to trigger the measurement of the second physiological signal based on the first signal from the first sensor.

2. The monitoring system according to claim 1, wherein said first signal is a physiological signal from said subject.

3. The monitoring system according to claim 2, wherein said first physiological signal or said data signal from said first sensor is one or more selected from heart rate (HR), blood pressure (BP), blood pH, respiration, such as respiration frequency, skin and/or core body temperature, snoring sound or other sounds of the subject, electromyography (EMG), submental EMG, galvanic skin response (GSR), electrocardiography (ECG), electroencephalography (EEG), phonocardiogram (PCG), arterial oxygen saturation ($SpO_2$), muscle activity, motion, emotions, arterial saturation of carbon monoxide (SpCO), sensors for physiological gases, such as a gas exhaled from the lungs, such as exhaled nitrogen oxide.

4. The monitoring system according to claim 1, wherein said first signal is a non-physiological signal obtained from one or more selected from a Global Positioning System (GPS), a pressure sensor, an accelerometer, air humidity, environment temperature, predetermined and specific radio signal or lack of the same, Radio Frequency Identification (RFID) tag, chemical or biochemical sensors, such as for toxic or hazardous gases, on-demand signal from the subject or another person responsible for monitoring the physiological signal from said subject.

5. The monitoring system according to any one of claims 1-2, wherein said first sensor is for the optical measurement based on photoplethysmography (PPG).

6. The monitoring system according to claim 5, wherein said first and/or second sensor is for optical measurements of one or more physiological signals selected from respiration, such as respiration frequency, heart rate (HR), arterial oxygen saturation by pulse oximetry ($SpO_2$), saturation of carbon monoxide (SpCO), methaemoglonin (metHb), heart rate variability, blood pressure, tissue perfusion, haemoglobin concentration.

7. The monitoring system according to any one of claims 1-3, wherein said first sensor is for measuring electric potentials.

8. The monitoring system according to any one of claims 1-2, wherein said first and/or second sensor is for measuring one or more physiological signal selected from electrocardiography (ECG), electromyography (EMG) electroencephalography (EEG), galvanic skin response (GSR), phonocardiogram (PCG), arterial oxygen saturation ($SpO_2$), muscle activity, emotions, arterial saturation of carbon monoxide (SpCO), blood carbon dioxide ($CO_2$) and different forms thereof, blood pH, blood pressure, respiration, such as respiration frequency, heart rate (HR), snoring sound or other sounds of the subject, and skin and/or core body temperature.

9. The monitoring system according to any one of claims 1-2, wherein said first sensor is for mechanical measurements for measuring one or more physiological parameter selected from respiration, such as respiration frequency, blood pressure, sweat production, tissue perfusion, function of heart, including its valves and vessels, and motion.

10. The monitoring system according to claim 9, wherein said mechanical measurements is selected from ultrasound based measurements and/or a phonocardiogram (PCG).

11. The monitoring system according to any one of claims 1-10, wherein said subject is a human being.

Fig. 1

Patch

Antenna

Electronics

Battery

Photo detector

LEDs

Fig. 2

Fig. 3

Fig. 4

(a) Adhesive patch

(b) Plastic housing - top

(c) Battery

(d) Plastic housing- middle

(e) Printed circuit board PCB

(f) Plastic housing - bottom

(g) Bio-compatible "window"

Fig. 5

Fig. 6

Fig. 7

Fig. 8

Fig. 9

Fig. 10

Fig. 11

Fig. 12

Fig. 13

Fig. 14

29

30

16 mm

Fig. 15

Adhesive patch

Battery frame

Battery

Top housing

PCB

Photodiode

Bottom housing

The disposable
part:
"Adhesive cap"

The reusable
part:
"Sensor house"

Europäisches
Patentamt

European
Patent Office

Office européen
des brevets

# EUROPEAN SEARCH REPORT

Application Number

EP 14 19 5630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | RASMUS G HAAHR ET AL: "A wearable ''electronic patch'' for wireless continuous monitoring of chronically diseased patients", MEDICAL DEVICES AND BIOSENSORS, 2008. ISSS-MDBS 2008. 5TH INTERNATIONAL SUMMER SCHOOL AND SYMPOSIUM ON, IEEE, PISCATAWAY, NJ, USA, 1 June 2008 (2008-06-01), pages 66-70, XP031290412, ISBN: 978-1-4244-2252-4 * title * * sections III and IV; the last paragraph of section IV * * figures 1-6 * * the whole document * | 1-11 | INV. A61B5/00 A61B5/024 A61B5/0245 A61B5/0488 A61B5/08 |
| Y | US 2009/076343 A1 (JAMES KRISTOFER J [US] ET AL) 19 March 2009 (2009-03-19) * figures 1-3 * * paragraphs [0024], [0029], [0032] - [0039], [0048], [0057], [0060], [0077], [0078], [0089], [0118] * * paragraphs [0132], [0133], [0137] - [0141], [0143], [0146], [0151], [0153], [0155] - [0158] * * paragraphs [0161], [0162], [0167] * | 1-11 | |
| Y,D | WO 2006/094513 A2 (COLOPLAST AS [DK]; DELTA [DK]; FAARBAEK SUSANNE HOLM [DK]; HOPPE KARST) 14 September 2006 (2006-09-14) * the whole document * | 1-11 | TECHNICAL FIELDS SEARCHED (IPC)<br><br>A61B |
| A | US 5 273 036 A (KRONBERG HARALD [DE] ET AL) 28 December 1993 (1993-12-28) * the whole document * | 1-11 | |

-/--

The present search report has been drawn up for all claims

| Place of search | Date of completion of the search | Examiner |
|---|---|---|
| Munich | 31 March 2015 | Olapinski, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 19 5630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2006/109072 A2 (HIDALGO LTD [GB]; PISANI JUSTIN [GB]; HOWARD PETER [GB]; CADE DANIEL [])<br>19 October 2006 (2006-10-19)<br>* page 15, lines 5-9, 22-26 *<br>* page 22, line 19 - page 23, line 3 *<br>* claims 84,96,97,112 *<br>----- | 1-11 | |

TECHNICAL FIELDS
SEARCHED      (IPC)

The present search report has been drawn up for all claims

| Place of search | Date of completion of the search | Examiner |
|---|---|---|
| Munich | 31 March 2015 | Olapinski, Michael |

EPO FORM 1503 03.82 (P04C01)

**ANNEX TO THE EUROPEAN SEARCH REPORT
ON EUROPEAN PATENT APPLICATION NO.**                    EP 14 19 5630

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2015

| Patent document cited in search report | Publication date | Patent family member(s) | Publication date |
|---|---|---|---|
| US 2009076343 A1 | 19-03-2009 | US 2009073991 A1<br>US 2009076343 A1<br>US 2009076346 A1<br>WO 2009036316 A1<br>WO 2009036333 A1 | 19-03-2009<br>19-03-2009<br>19-03-2009<br>19-03-2009<br>19-03-2009 |
| WO 2006094513 A2 | 14-09-2006 | AU 2006222414 A1<br>CA 2600427 A1<br>CN 101779949 A<br>EP 1871218 A2<br>EP 2412306 A2<br>HK 1118689 A1<br>JP 5086235 B2<br>JP 5665139 B2<br>JP 2008532596 A<br>JP 2012135626 A<br>US 2008275327 A1<br>US 2014288381 A1<br>WO 2006094513 A2 | 14-09-2006<br>14-09-2006<br>21-07-2010<br>02-01-2008<br>01-02-2012<br>02-02-2011<br>28-11-2012<br>04-02-2015<br>21-08-2008<br>19-07-2012<br>06-11-2008<br>25-09-2014<br>14-09-2006 |
| US 5273036 A | 28-12-1993 | NONE | |
| WO 2006109072 A2 | 19-10-2006 | AU 2006235722 A1<br>CA 2650576 A1<br>EP 1890589 A2<br>EP 2420185 A2<br>JP 2009500047 A<br>JP 2013013747 A<br>JP 2014237015 A<br>US 2010063365 A1<br>US 2013237772 A1<br>WO 2006109072 A2 | 19-10-2006<br>19-10-2006<br>27-02-2008<br>22-02-2012<br>08-01-2009<br>24-01-2013<br>18-12-2014<br>11-03-2010<br>12-09-2013<br>19-10-2006 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006094513 A
- WO 03065926 A
- US 5273036 A
- US 5458124 A
- US 6372951 B
- US 6385473 B
- WO 9959465 A
- US 5054488 A
- US 20090076343 A
- WO 2006109072 A
- WO 03039340 A
- US 6701170 B
- US 7079880 B
- US 7343187 B

**Non-patent literature cited in the description**

- **RASMUS G. HAAHR et al.** Proceedings of the 5th International Workshop on Wearable and Implantable Body Sensor Networks. *conjunction with The 5th International Summer School and Symposium on Medical Devices and Biosensors The Chinese University of Hong Kong,* 01 June 2008
- **RASMUS G. HAAHR et al.** *Proceedings of the 29th Annual International Conference of the IEEE,* 23 August 2007
- **TEARNEY, G. J. et al.** Determination of the refractive index of highly scattering human tissue by optical coherence tomography. *Opt Lett,* 1995, vol. 20, 2258
- **DING, H. et al.** Refractive indices of human skin tissues at eight wavelengths and estimated dispersion relations between 300 and 1600 nm. *Phys Med Biol,* March 2006, vol. 51 (6), 1479-1489
- **DUUN et al.** *Jour. Micromech. Microeng.,* 2010, vol. 20